# EUROPÄISCHE PATENTSCHRIFT

(11) **EP 3 695 809 B1**
(45) Veröffentlichungstag und Bekanntmachung des Hinweises auf die Patenterteilung: **23.10.2024**
(21) Anmeldenummer: 20150832.2
(22) Anmeldetag: 09.01.2020
(51) Int. Cl.: A61F 2/16, A61B 17/30

(54) **OPHTHALMOLOGISCHES HANDGERÄT**
OPHTHALMOLOGICAL HAND-HELD DEVICE
APPAREIL OPHTALMOLOGIQUE PORTATIF

(30) Priorität: 13.02.2019 DE 102019201833
(43) Veröffentlichungstag der Anmeldung: 19.08.2020
(73) Patentinhaber: Geuder AG, 69126 Heidelberg (DE)
(72) Erfinder: Eder, Jürgen, 69251 Gaiberg (DE); Amon, Michael, 1020 Wien (DE)
(74) Vertreter: Ullrich & Naumann PartG mbB

(56) Entgegenhaltungen:
- EP-A2- 1 285 631
- WO-A1-2014/172503
- WO-A2-2007/106813
- US-A1- 2003 088 253

## Beschreibung

Die Erfindung betrifft ein ophthalmologisches Handgerät mit einem Grundkörper und einem mit dem Grundkörper verbundenen Führungsrohr, wobei ein distales Ende des Führungsrohrs scharf und/oder spitz ausgebildet ist, sodass dieses in ein Auge einstechbar ist.

Ophthalmologische Handgeräte der in Rede stehenden Art werden bei unterschiedlichsten augenchirurgischen Eingriffen verwendet. Beispielsweise handelt es sich dabei um Kanülen, die in das Auge eingestochen werden, um in dem Augeninneren zu manipulieren.

Bei einem solchen augenchirurgischen Eingriff kann es sich um die Implantation einer Intraokularlinse handeln. Dabei ist es mitunter notwendig, die Haptik der Intraokularlinse jeweils durch einen sogenannten Skleratunnel zu führen und damit die Intraokularlinse zu fixieren. Hierbei wird zunächst eine Kanüle in das Auge eingestochen, um den Skleratunnel zu bilden. Mit einem zweiten Instrument wird das Ende der Haptik in die Kanüle eingeschoben, sodass die Haptik mit der Kanüle durch den Skleratunnel aus dem Auge gezogen werden kann. An dieser Stelle kann sodann die Haptik festgelegt werden.

Bei den bekannten Vorrichtungen ist es somit nachteilig, dass stets neben der Kanüle mindestens ein weiteres Instrument in das Auge eingeführt werden muss, um ein Objekt in dem Auge - beispielsweise die Haptik einer Intraokularlinse - in das Kanülenende einzubringen. Somit sind mehrere Öffnungen an dem Auge anzubringen, was ein erhöhtes Infektionsrisiko bedeutet und des Weiteren den Heilungsprozess verlängert. Weiterhin ist die Handhabung mehrerer Instrumente für den Operateur aufwändig, sodass hohe Anforderungen an das Geschick des Operators gestellt werden und der Eingriff relativ lange dauert.

US 2003/0088253 offenbart ein Ophthalmologisches Handgerät zur Extraktion von Intraokularlinsen. Das Handgerät weist ein Führungsrohr auf, wobei ein distales Ende des Führungsrohrs scharf ausgebildet ist, wobei eine Greifeinrichtung verschiebbar in dem Führungsrohr angeordnet ist.

EP1285631 offenbart ein Ophthalmologisches Handgerät für die Netzhautchirurgie. Das Handgerät weist ein Führungsrohr auf, wobei eine Greifeinrichtung verschiebbar in dem Führungsrohr angeordnet ist.

Der vorliegenden Erfindung liegt daher die Aufgabe zugrunde, ein ophthalmologisches Handgerät der eingangs genannten Art derart auszugestalten und weiterzubilden, dass mit konstruktiv einfachen Mitteln die Handhabung verbessert wird.

Erfindungsgemäß wird die voranstehende Aufgabe durch die Merkmale des Anspruches 1 gelöst. Danach ist das in Rede stehende ophthalmologische Handgerät dadurch gekennzeichnet, dass eine Greifeinrichtung verschiebbar in dem Führungsrohr angeordnet ist und dass die Greifeinrichtung aus dem Führungsrohr ausbringbar ist, um ein Objekt zu ergreifen.

In erfindungsgemäßer Weise ist erkannt worden, dass durch die Kombination eines Führungsrohrs mit einem distalen Ende, welches scharf und/oder spitz ausgebildet ist und somit als Kanüle dienen kann, und einer Greifeinrichtung, mit welcher ein Objekt in dem Auge ergriffen werden kann, die zu Grunde liegende Aufgabe in überraschend einfacher Weise lösbar ist. Im Konkreten handelt es sich bei dem erfindungsgemäßen ophthalmologischen Handgerät um eine Kombination aus einer in das Auge einstechbaren Kanüle und einer Greifeinrichtung. Somit kann der Operateur zunächst das distale Ende des Führungsrohrs in das Auge einstechen und sodann über die in dem Führungsrohr angeordnete Greifeinrichtung die notwendige Manipulation innerhalb des Auges vornehmen. Somit ist lediglich eine einzige Inzision an dem Auge notwendig und kann der Operateur einhändig ein Objekt ergreifen. Im Konkreten könnte die Greifeinrichtung über ein Betätigungsmittel innerhalb des Führungsrohres verschoben bzw. bewegt werden. Auch ist es denkbar, dass ein von der Greifeinrichtung ergriffenes Objekt - bspw. eine Haptik - zumindest teilweise in das Führungsrohr hineingezogen werden kann.

In vorteilhafter Weise ist die die Greifeinrichtung vollständig in das Führungsrohr einbringbar bzw. einziehbar. Somit kann das distale Ende des Führungsrohrs in idealer Weise zum Einstechen verwendet werden, ohne dass die Greifeinrichtung hierbei stört. Des Weiteren wird vermieden, dass die Greifeinrichtung beim Einstechen in das Auge beschädigt wird.

In weiter vorteilhafter Weise kann der Grundkörper mit einem Handgriff verbunden sein. Dabei ist es des Weiteren denkbar, dass der Körper lösbar mit dem Handgriff verbunden ist. Somit könnte der Grundkörper als Einweginstrument ausgebildet sein und der Handgriff sterilisierbar sein. Alternativ könnten der Grundkörper und der Handgriff unlösbar miteinander verbunden bzw. einteilig ausgebildet sein.

Gemäß einer weiteren vorteilhaften Ausgestaltung könnte der Handgriff als Betätigungsmittel dienen, um die Greifeinrichtung aus dem Führungsrohr auszubringen und/oder in das Führungsrohr einzubringen bzw. einzuziehen. Somit kann der Operateur bspw. durch ein Zusammendrücken des Handgriffs die Greifeinrichtung aus dem Führungsrohr ausbringen bzw. in dieses einziehen.

Im Konkreten ist es denkbar, dass die Greifeinrichtung bei nicht betätigtem Handgriff aus dem Führungsrohr hinausragt und durch die Betätigung des Handgriffs in das Führungsrohr einbringbar ist bzw. eingezogen wird. Folglich könnte der Operateur zunächst mit betätigtem Handgriff das Führungsrohr in das Auge einstechen, wobei die Greifeinrichtung sich innerhalb des Führungsrohrs befindet. Sobald das Führungsrohr in das Auge eingestochen ist, kann der Chirurg den Handgriff freigeben, sodass die Greifeinrichtung aus dem Führungsrohr hinausgeschoben wird, um ein Objekt innerhalb des Auges zu ergreifen. Das Objekt könnte sodann durch eine Betätigung des Betätigungsmittels zumindest geringfügig in das Führungsrohr eingezogen werden. Auch ist es möglich, dass die Greifeinrichtung bei nicht aktiviertem Betätigungsmittel bzw. Handgriff zumindest im Wesentlichen in dem Führungsrohr angeordnet ist und durch Betätigung des Betätigungsmittels bzw. des Handgriffs aus dem Führungsrohr ausgebracht wird.

In weiter vorteilhafter Weise könnte die Greifeinrichtung zwei miteinander zusammenwirkende Greifarme aufweisen. Die Greifarme könnten pinzettenartig ausgebildet sein bzw. Klemmbacken aufweisen. Dabei ist für die zugrundeliegende Offenbarung der Begriff "greifen" im weitesten Sinne zu verstehen. Beispielsweise könnte zumindest einer der Greifarme eine Schneide aufweisen, sodass die Greifeinrichtung auch zum Schneiden dienen könnte.

Des Weiteren ist es denkbar, dass das Führungsrohr abgewinkelt ausgebildet ist. Eine solche Konstruktion kann für Eingriffe in Regionen des Auges vorteilhaft sein, die mit einem nicht abgewinkelten Führungsrohr nur schwer zu erreichen wären. Alternativ ist es denkbar, dass das Führungsrohr gerade ausgebildet ist. Eine solche Konstruktion zeichnet sich durch ihre einfache Herstellung aus.

In besonders vorteilhafter Weise kann das Führungsrohr einen Durchmesser von kleiner oder gleich 30 Gauge aufweisen, vorzugsweise einen Durchmesser von kleiner oder gleich 27 Gauge. Eine entsprechende Dimensionierung erlaubt es, dass das Führungsrohr durch einen relativ kleinen Einstichkanal in das Auge einzubringen ist und ist des Weiteren ausreichend groß, um die Greifeinrichtung darin anzuordnen. Im Konkreten ist es denkbar, dass die Greifeinrichtung zum Eingreifen der Haptik einer Intraokularlinse dient. An dieser Stelle wird darauf hingewiesen, dass eine Intraokularlinse üblicherweise eine zentrale optische Linse aufweist, an der sich peripher eine Haptik anschließt, die die optische Linse im Auge fixiert. Die erfindungsgemäße Vorrichtung eignet sich in idealer Weise zur Fixierung der Haptik einer Intraokularlinse in einem Skleratunnel. Der Skleratunnel kann durch das Einstechen des Führungsrohrs in das Auge erzeugt werden, wobei die in dem Auge befindliche Haptik durch die Greifeinrichtung in die Öffnung an dem distalen Ende des Führungsrohrs eingezogen werden kann.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung, eine Seitenansicht eines erfindungsgemäßen ophthalmologischen Handgeräts,
- Fig. 2: in einer schematischen, perspektivischen Darstellung das distale Ende eines erfindungsgemäßen ophthalmologischen Handgeräts,
- Fig. 3: in einer schematischen Darstellung eine Seitenansicht des distalen Endes eines erfindungsgemäßen ophthalmologischen Handgeräts gemäß Fig. 2,
- Fig. 4: in einer weiteren schematischen Darstellung eine Seitenansicht des distalen Endes eines erfindungsgemäßen ophthalmologischen Handgeräts gemäß Fig. 2, und
- Fig. 5: in einer weiteren schematischen, perspektivischen Darstellung das distale Ende eines erfindungsgemäßen ophthalmologischen Handgeräts gemäß Fig. 2.

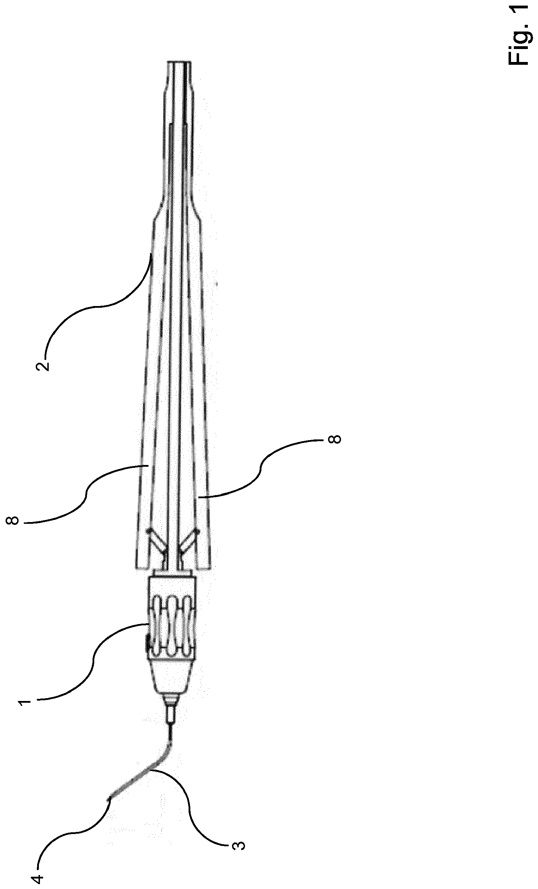

Fig. 1 zeigt in einer schematischen Darstellung, eine Seitenansicht eines erfindungsgemäßen ophthalmologischen Handgeräts. Das Handgerät weist einen Grundkörper 1 auf, der an einem Handgriff 2 angeordnet ist. Der Handgriff umfasst zwei Griffelemente 8. Von dem Grundkörper 1 erstreckt sich ein Führungsrohr 3, dessen distales Ende 4 scharf bzw. spitz ausgebildet ist, sodass dieses in ein Auge, beispielsweise durch die Bindehaut in den vorderen Augenabschnitt hinein, einstechbar ist. Das Führungsrohr 3 ist somit als Kanüle verwendbar. In dem hier dargestellten Ausführungsbeispiel ist das Führungsrohr 3 abgewickelt ausgebildet. Es ist jedoch denkbar, dass das Führungsrohr 3 gerade ausgestaltet ist.

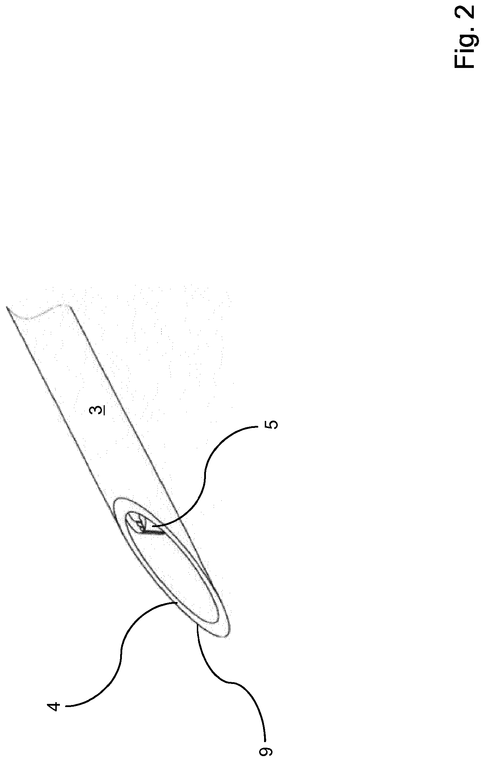

Die Fig. 2 bis 5 zeigen - vergrößert - das distale Ende 4 des erfindungsgemäßen Handgeräts. Dabei ist deutlich zu erkennen, dass in dem Führungsrohr 3 eine Greifeinrichtung 5 verschiebbar angeordnet ist. Bei einem Herausschieben der Greifeinrichtung 5 aus den Führungsrohr 3 werden die Greifarme 6 geöffnet, sodass ein Objekt 7, beispielsweise die Haptik einer Intraokularlinse, von den Greifarmen 6 ergriffen werden kann. Beim Zurückziehen der Greifarme 6 in das Führungsrohr 3 hinein werden die Greifarme 6 gegeneinandergedrückt, sodass das Objekt 7 ergriffen und ggf. in das Führungsrohr 3 eingezogen werden kann.

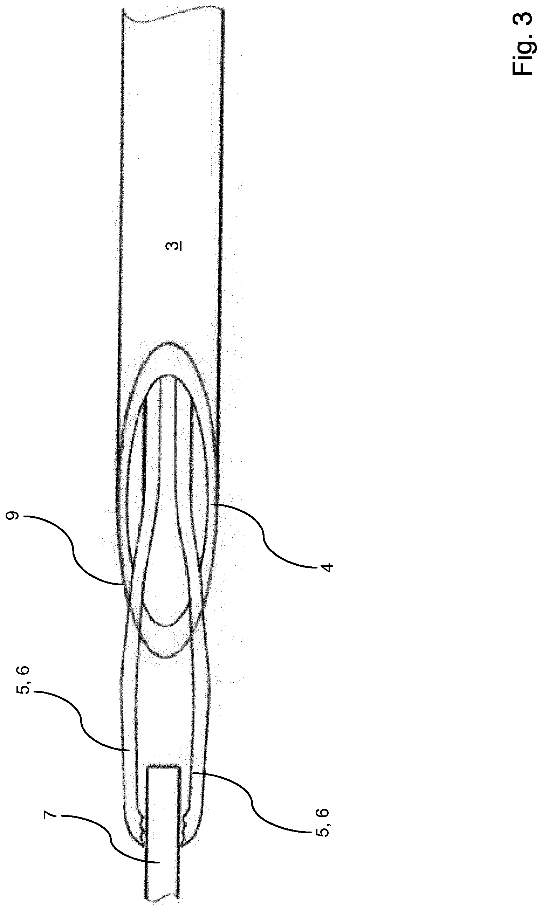

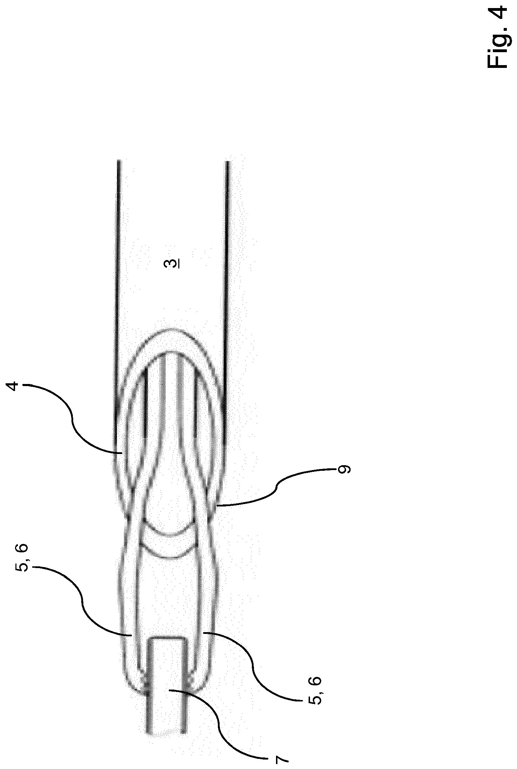

In dem hier dargestellten Ausführungsbeispiel dient der Handgriff 2 als Betätigungsmittel zum Verschieben der Greifeinrichtung 5 innerhalb des Führungsrohrs 3. Hierzu kann der Handgriff zwei Griffelemente 8 aufweisen, die beispielsweise über eine Feder vorgespannt sind. Sofern die Griffelemente 8 gegeneinandergedrückt sind, kann die Greifeinrichtung 5 zumindest im Wesentlichen vollständig innerhalb des Führungsrohr 3 angeordnet sein (vgl. Fig. 2). In diesem Zustand kann das distale Ende 4 in das Auge eingestochen werden, ohne dass die Greifeinrichtung 5 das Auge verletzt oder durch Augengewebe beschädigt wird. Nachdem das distale Ende 4 in das Auge eingeführt ist, kann der Chirurg die Griffelemente 8 freigeben, so dass die Greifarme 6 aus dem distalen Ende 4 herausgeschoben werden, um ein Objekt 7 zu erfassen (vgl. Fig. 3 und 4).

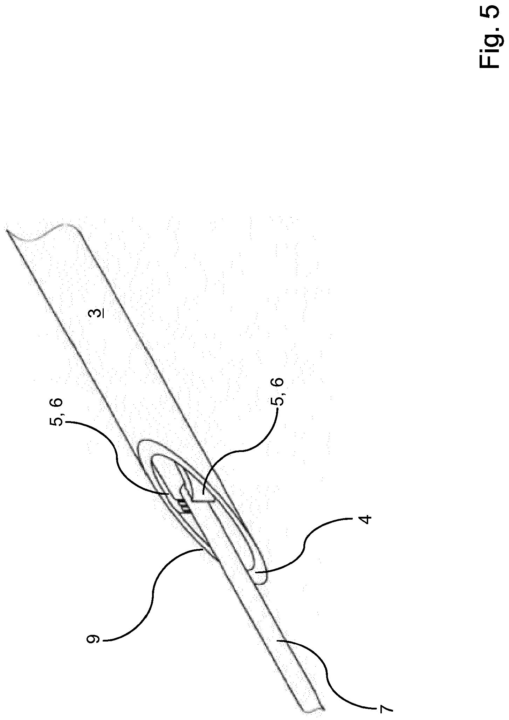

Wenn sich das Objekt 7 zwischen den Greifarmen 6 befindet, können die Griffelemente 8 zusammengedrückt werden, so dass die Greifarme 6 zusammen mit dem Objekt 7 in das Führungsrohr 4 verlagert werden (vgl. Fig. 5). In diesem Zustand kann das distale Ende 4 zusammen mit dem Objekt 7 aus dem Auge gezogen werden. Somit wird das Objekt 7, beispielsweise die Haptik einer Intraokularlinse, durch den Skleratunnel gezogen. Es wird darauf hingewiesen, dass es auch möglich ist, dass die Greifeinrichtung 5 bei nicht betätigtem Handgriff 2 innerhalb des Führungsrohrs 4 angeordnet ist und durch ein Zusammendrücken der Griffelemente 8 aus dem Führungsrohr 4 herausgeschoben wird.

Um das distale Ende 4 möglichst einfach in das Auge einstechen zu können, ist es denkbar, dass die äußeren Kanten 9 des distalen Endes 4 als scharfe Schneidkanten realisiert sind. Des Weiteren ist es denkbar, dass der Grundkörper 1 von dem Handgriff 2 abgenommen werden kann oder fest bzw. einteilig mit dem Grundkörper 1 ausgebildet ist.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken.

### Bezugszeichenliste

- 1: Grundkörper
- 2: Handgriff
- 3: Führungsrohr
- 4: distales Ende (Führungsrohr)
- 5: Greifeinrichtung
- 6: Greifarme
- 7: Objekt
- 8: Griffelement
- 9: äußere Kante (distales Ende)

## Patentansprüche

1. Ophthalmologisches Handgerät mit einem Grundkörper (1) und einem mit dem Grundkörper (1) verbundenen Führungsrohr (4), wobei eine Greifeinrichtung (5) verschiebbar in dem Führungsrohr (4) angeordnet ist und dass die Greifeinrichtung (5) aus dem Führungsrohr (4) ausbringbar ist, um ein Objekt (7) zu ergreifen,
**dadurch gekennzeichnet,**
**dass** ein distales Ende (4) des Führungsrohrs (4) scharf und/oder spitz ausgebildet ist, so dass dieses in ein Auge einstechbar ist.

2. Ophthalmologisches Handgerät nach Anspruch 1, **dadurch gekennzeichnet, dass** die Greifeinrichtung (5) vollständig in das Führungsrohr (4) einbringbar ist.

3. Ophthalmologisches Handgerät nach Anspruch 1 oder 2, **dadurch gekennzeichnet, dass** der Grundkörper (1), vorzugsweise lösbar, mit einem Handgriff (2) verbunden ist.

4. Ophthalmologisches Handgerät nach Anspruch 3, **dadurch gekennzeichnet, dass** der Handgriff (2) als Betätigungsmittel dient, um die Greifeinrichtung (5) aus dem Führungsrohr (4) auszubringen und/oder in das Führungsrohr (4) einzubringen.

5. Ophthalmologisches Handgerät nach Anspruch 4, **dadurch gekennzeichnet, dass** die Greifeinrichtung (5) bei nicht betätigtem Handgriff (2) aus dem Führungsrohr (4) hinausragt und durch Betätigung des Handgriffs (2) in das Führungsrohr (4) einbringbar ist.

6. Ophthalmologisches Handgerät nach einem der Ansprüche 1 bis 5, **dadurch gekennzeichnet, dass** die Greifeinrichtung (5) zwei miteinander zusammenwirkende Greifarme (6) aufweist.

7. Ophthalmologisches Handgerät nach einem der Ansprüche 1 bis 6, **dadurch gekennzeichnet, dass** das Führungsrohr (4) abgewinkelt ausgebildet ist.

8. Ophthalmologisches Handgerät nach einem der Ansprüche 1 bis 6, **dadurch gekennzeichnet, dass** das Führungsrohr (4) gerade ausgebildet ist.

9. Ophthalmologisches Handgerät nach einem der Ansprüche 1 bis 8, **dadurch gekennzeichnet, dass** das Führungsrohr (4) einen Durchmesser von kleiner oder gleich 30 Gauge, vorzugsweise von kleiner oder gleich 27 Gauge aufweist.

10. Ophthalmologisches Handgerät nach einem der Ansprüche 1 bis 9, **dadurch gekennzeichnet, dass** die Greifeinrichtung (5) zum Ergreifen der Haptik einer Intraokularlinse dient.

## Claims

1. Ophthalmological hand-held device having a base member (1) and a guide pipe (4) which is connected to the base member (1), wherein a gripping device (5) is displaceably arranged in the guide pipe (4) and the gripping device (5) can be removed from the guide pipe (4) in order to grip an object (7),
**characterised in that** a distal end (4) of the guide pipe (4) is constructed to be sharp and/or pointed so that it can be inserted into an eye.

2. Ophthalmological hand-held device according to claim 1, **characterised in that** the gripping device (5) can be introduced completely into the guide pipe (4).

3. Ophthalmological hand-held device according to claim 1 or 2, **characterised in that** the base member (1) is preferably releasably connected to a handle (2).

4. Ophthalmological hand-held device according to claim 3, **characterised in that** the handle (2) acts as an activation means in order to remove the gripping device (5) from the guide pipe (4) and/or to introduce it into the guide pipe (4) .

5. Ophthalmological hand-held device according to claim 4, **characterised in that**, when the handle (2) is not activated, the gripping device (5) protrudes from the guide pipe (4) and, by activating the handle (2), can be introduced into the guide pipe (4).

6. Ophthalmological hand-held device according to any one of claims 1 to 5, **characterised in that** the gripping device (5) has two gripping arms (6) which cooperate with each other.

7. Ophthalmological hand-held device according to any one of claims 1 to 6, **characterised in that** the guide pipe (4) is constructed in an angled manner.

8. Ophthalmological hand-held device according to any one of claims 1 to 6, **characterised in that** the guide pipe (4) is constructed in a linear manner.

9. Ophthalmological hand-held device according to any one of claims 1 to 8, **characterised in that** the guide pipe (4) has a diameter of less than or equal to 30 Gauge, preferably of less than or equal to 27 Gauge.

10. Ophthalmological hand-held device according to any one of claims 1 to 9, **characterised in that** the gripping device (5) is used to grip the haptics of an intraocular lens.

## Revendications

1. Appareil ophtalmologique portatif avec un corps de base (1) et un tube de guidage (4) relié au corps de base (1), dans lequel un dispositif de préhension (5) est disposé de manière coulissante dans le tube de guidage (4) et en ce que le dispositif de préhension (5) peut être extrait du tube de guidage (4) afin de saisir un objet (7),
**caractérisé en ce que**
une extrémité distale (4) du tube de guidage (4) est tranchant et/ou pointu, de sorte qu'il peut être introduit dans un oeil.

2. Appareil ophtalmologique portatif selon la revendication 1, **caractérisé en ce que** le dispositif de préhension (5) peut être introduit entièrement dans le tube de guidage (4).

3. Appareil ophtalmologique portatif selon la revendication 1 ou 2, **caractérisé en ce que** le corps de base (1) est relié, de préférence de manière amovible, avec une poignée (2).

4. Appareil ophtalmologique portatif selon la revendication 3, **caractérisé en ce que** la poignée (2) sert de moyen d'actionnement afin d'extraire le dispositif de préhension (5) hors du tube de guidage (4) et/ou de l'introduire dans le tube de guidage (4).

5. Appareil ophtalmologique portatif selon la revendication 4, **caractérisé en ce que** le dispositif de préhension (5) dépasse du tube de guidage (4) lorsque la poignée (2) n'est pas actionnée et peut être introduit dans le tube de guidage (4) grâce à l'actionnement de la poignée (2).

6. Appareil ophtalmologique portatif selon l'une des revendications 1 à 5, **caractérisé en ce que** le dispositif de préhension (5) comprend deux bras de préhension (6) en interaction l'un avec l'autre.

7. Appareil ophtalmologique portatif selon l'une des revendications 1 à 6, **caractérisé en ce que** le tube de guidage (4) est coudé.

8. Appareil ophtalmologique portatif selon l'une des revendications 1 à 6, **caractérisé en ce que** le tube de guidage (4) est droit.

9. Appareil ophtalmologique portatif selon l'une des revendications 1 à 8, **caractérisé en ce que** le tube de guidage (4) présente un diamètre inférieur ou égal au calibre 30, de préférence inférieur ou égal au calibre 27.

10. Appareil ophtalmologique portatif selon l'une des revendications 1 à 9, **caractérisé en ce que** le dispositif de préhension (5) sert à saisir l'haptique d'une lentille intra-oculaire.
